# EUROPÄISCHE PATENTSCHRIFT

(11) **EP 1 372 460 B1**
(45) Veröffentlichungstag und Bekanntmachung des Hinweises auf die Patenterteilung: **22.09.2004**
(21) Anmeldenummer: 02787666.3
(22) Anmeldetag: 13.11.2002
(51) Int. Cl.: A61B 1/00, G02B 23/24

(54) **VORRICHTUNG ZUM POSITIONIEREN ZUMINDEST EINES OPTISCHEN BAUELEMENTS INNERHALB EINES ENDOSKOPISCHEN SYSTEMS**
DEVICE FOR POSITIONING AT LEAST ONE OPTICAL COMPONENT INSIDE AN ENDOSCOPIC SYSTEM
DISPOSITIF DE POSITIONNEMENT D'AU MOINS UN COMPOSANT OPTIQUE A L'INTERIEUR D'UN SYSTEME ENDOSCOPIQUE

(30) Priorität: 19.11.2001 DE 10157075
(43) Veröffentlichungstag der Anmeldung: 02.01.2004
(73) Patentinhaber: Karl Storz GmbH & Co. KG, 78532 Tuttlingen (DE)
(72) Erfinder: KEHR, Ulrich, 70771 Leinfelden-Echterdingen (DE); HÖFIG, Siegfried, 78570 Mühlheim (DE)
(74) Vertreter: Heuckeroth, Volker, Dr.
(86) Internationale Anmeldenummer: PCT/EP2002/012705
(87) Internationale Veröffentlichungsnummer: WO 2003/043489

(56) Entgegenhaltungen:
- DE-A- 2 328 595
- DE-A- 19 713 276
- US-A- 2 987 960
- US-A- 4 862 199
- US-A- 5 978 161

## Beschreibung

Die Erfindung betrifft eine Vorrichtung zum Positionieren zumindest eines optischen Bauelements innerhalb eines endoskopischen Systems, mit einem Gehäuse, durch das eine optische Achse des endoskopischen Systems verläuft, und in dem das zumindest eine Bauelement angeordnet ist, das um eine im wesentlichen parallel zu einer Längsachse des Gehäuses verlaufende Schwenkachse in den Strahlengang einschwenkbar und aus dem Strahlengang wieder ausschwenkbar ist, wobei das zumindest eine Bauelement an einem um die Schwenkachse verschwenkbaren Träger angeordnet ist.

Eine derartige Vorrichtung ist aus der DE 197 13 276 Al bekannt.

Unter einem optischen Bauelement werden im Sinne der vorliegenden Erfindung bspw. Linsen, Filter, Blenden, Polarisatoren und dergleichen verstanden, die in einer Endoskopoptik verwendet werden können. Ohne Beschränkung der Allgemeinheit kann ein optisches Bauelement auch eine Baugruppe aus den zuvor genannten Elementen umfassen.

Ein endoskopisches System kann im Sinne der vorliegenden Erfindung bspw. ein Endoskop sein, in das die eingangs genannte Vorrichtung integriert ist.

Der Strahlengang des endoskopischen Systems, der sich entlang der optischen Achse ausbreitet, kann der Strahlengang von Beleuchtungslicht, das sich von proximal nach distal ausbreitet, und/oder der Strahlengang von Beobachtungslicht sein, das sich von distal nach proximal ausbreitet.

Ein spezieller Anwendungsfall der vorliegenden Erfindung ist die Verwendung einer eingangs genannten Vorrichtung in einem Endoskop für die photodynamische Diagnose, die photodynamische Therapie oder für die Fluoreszenzdiagnose. Bei diesem speziellen Anwendungsfall ist das zumindest eine optische Bauelement üblicherweise ein Farbfilter, um eine kontrastreiche Beobachtung des zu untersuchenden Gewebes frei von einer die Beobachtung störenden Hintergrundstrahlung des Anregungslichtes, die das Beobachtungslicht überlagert, zu ermöglichen. Darüber hinaus ist auch bei diesem speziellen Anwendungsfall der eingangs genannten Vorrichtung eine herkömmliche Beobachtung des Gewebebereiches mit Weißlicht erwünscht, so daß solche Farbfilter nicht nur möglichst einfach in den Strahlengang einbringbar, sondern auch wieder herausnehmbar sein sollten.

Die aus der eingangs genannten DE 197 13 276 Al bekannte Vorrichtung zum Positionieren von Bauelementen innerhalb endoskopischer Systeme weist in einem Ausführungsbeispiel ein Revolverrad auf, das in dem Gehäuse der Vorrichtung um die Längsmittelachse des Gehäuses drehbar ist. Das Revolverrad trägt in einer Ebene verteilt drei optische Bauelemente, die um die Längsachse des Gehäuses als Schwenkachse in den Strahlengang des endoskopischen Systems ein- und.wieder ausgeschwenkt werden können. Die Längsachse des Gehäuses verläuft dabei parallel zur optischen Achse, ist von dieser jedoch beabstandet. Dies ist jedoch nachteilig, insbesondere bei einem Endoskop, da bei einem Endoskop die optische Achse mit der Längsmittelachse des mitunter dünnen Schafts des Endoskops zusammenfällt. Dies bedeutet bei der bekannten Vorrichtung, daß das Gehäuse der Vorrichtung nicht konzentrisch bzw. symmetrisch zur Längsachse des Schafts angeordnet werden kann, sondern den Schaft zu einer Seite hin weiter überragt als zur gegenüberliegenden Seite.

Würde man diese bekannte Anordnung dahingehend abändern, daß das Gehäuse der Vorrichtung die optische Achse des endoskopischen Systems konzentrisch bzw. symmetrisch umgibt, müßte bei unverändertem Durchmesser des Revolverrades, der wegen der vorgegebenen Größe der optischen Bauelemente nicht verkleinert werden kann, das Gehäuse im Durchmesser etwa auf das 1,5-fache vergrößert werden, wodurch der Nachteil einer quer zur Längsachse sehr platzgreifenden Vorrichtung bestehen würde.

Der Abstand eines jeweiligen Mittelpunktes der optischen Bauelemente zur Schwenkachse ist aufgrund der Ausgestaltung des Trägers als Revolverrad nachteiligerweise relativ klein, so daß der vom Bediener der Vorrichtung aufzubringende Verstellweg eines Stellgliedes sehr groß ist, um das jeweilige Bauelement in den Strahlengang ein- und wieder auszuschwenken. Dieser Nachteil wird noch dadurch verstärkt, daß der Abstand eines an dem Revolverrad vorgesehen Mitnehmerelements zur Schwenkachse demgegenüber relativ groß ist, da dieser Abstand einen gewissen minimalen Betrag nicht unterschreiten kann, da sonst der Materialsteg des Revolverrades zwischen zwei benachbarten Bauelementen zu schmal wird. Somit muß das Stellglied einen verhältnismäßig großen Weg zurücklegen, um das Revolverrad von einem Zustand in den nächsten zu schalten, was für ein schnelles und komfortables Umschalten der Vorrichtung hinderlich ist.

Um diesem Nachteil abzuhelfen, ist in der DE 199 03 437 Al vorgeschlagen worden, das zumindest eine optische Bauelement an einem L-förmigen Träger anzuordnen, der um eine quer zur Längsachse des Gehäuses und damit quer zur optischen Achse des endoskopischen Systems verlaufende Schwenkachse verschwenkbar ist. Durch diese Ausgestaltung der Vorrichtung wird gegenüber der zuvor genannten bekannten Vorrichtung eine geringere Abmessung des Gehäuses quer zur Längsachse des Gehäuses erreicht. Bei dieser bekannten Vorrichtung ergibt sich jedoch ein anderer Nachteil. Für das zumindest eine in den Strahlengang ein- und ausschwenkbare Bauelement ist üblicherweise ein sich entsprechend der Dicke des optischen Bauelements in Richtung der Längsachse erstreckender Spalt vorgesehen. Dieser Spalt ist in axialer Richtung häufig von anderen Bauelementen des endoskopischen Systems, bspw. Abbildungslinsen oder Blenden eingegrenzt.

Der Spalt für das ein- und auszuschwenkende Bauelement sollte in Richtung der optischen Achse möglichst nur geringfügig größer als die axiale Abmessung dieses Bauelements sein. Aufgrund der Verschwenkbarkeit des zumindest einen Bauelements um eine quer zur optischen Achse verlaufende Schwenkachse und des damit verbundenen Schwenkbereichs benötigt das zumindest eine Bauelement jedoch eine Spaltbreite in axialer Richtung, die die axiale Abmessung des Bauelements deutlich überschreitet. Der Nachteil dieser bekannten Vorrichtung besteht somit aufgrund des erheblichen Schwenkbereichs des Bauelements in einer konstruktiven Einschränkung des optischen Systems.

Der Erfindung liegt daher die Aufgabe zugrunde, eine Vorrichtung der eingangs genannten Art dahingehend weiterzubilden, daß die vorstehend genannten Nachteile vermieden werden, daß insbesondere das Gehäuse der Vorrichtung in Abhängigkeit von der Abmessung des zumindest einen Bauelements möglichst kleinbauend ausgestaltet werden kann, und sich ein geringer Verstellweg eines Betätigungselements zum Ein- und Ausschwenken des zumindest einen Bauelements ergibt.

Erfindungsgemäß wird diese Aufgabe hinsichtlich der eingangs genannten Vorrichtung durch eine Vorrichtung gemäß Anspruch 1 gelöst; im einzelnen dadurch, daß der kleinste Abstand einer Innenwand des Gehäuses von der Schwenkachse kleiner ist als der größte Abstand der Schwenkachse zu einer Außenkante des zumindest einen Bauelements.

Bei der erfindungsgemäßen Vorrichtung, bei der das zumindest eine optische Bauelement wie bei der eingangs genannten bekannten Vorrichtung um eine parallel zur Längsachse des Gehäuses verlaufende Schwenkachse verschwenkbar ist, ist das Revolverrad durch einen Träger ersetzt, der die Nachteile des Revolverrades vermeidet, indem ein kleinster Abstand einer Innenwand des Gehäuses von der Schwenkachse kleiner ist als ein größter Abstand der Schwenkachse zu einer Außenkante des zumindest einen Bauelements. Bei der erfindungsgemäßen Vorrichtung befindet sich demnach die Schwenkachse des zumindest einen Bauelements im Unterschied zu dem bekannten Revolverrad näher an der Gehäuseinnenwand, wodurch sich auch etwaige Mitnehmerelemente einer Betätigungseinrichtung zum einen nahe an der Gehäuseinnenwand, was zu einer konstruktiv einfachen Betätigungsvorrichtung führt, und zugleich nahe an der Schwenkachse anordnen lassen, wodurch sich ein geringer Betätigungsweg zum Ein- und Ausschwenken des zumindest einen Bauelements ergibt. Da das zumindest eine Bauelement um eine parallel zur optischen Achse verlaufende Schwenkachse in den Strahlengang ein- und ausschwenkbar ist, kann auch der für dieses Bauelement vorgesehene Spalt in Richtung der Längsachse so schmal ausgebildet werden, wie es gerade der axialen Abmessung bzw. Dicke des optischen Bauelements entspricht.

In einer bevorzugten Ausgestaltung ist eine Abmessung des Innenraums des Gehäuses von der Längsachse zur Innenwand des Gehäuses in der Schwenkebene des zumindest einen Bauelements etwa 1,5 bis 2 Mal so groß wie die größte Abmessung des zumindest einen Bauelements in der Schwenkebene.

Für diese Maßnahme wird eine in Abhängigkeit der Abmessung des zumindest einen Bauelements optimal minimierte Baugröße des Gehäuses der Vorrichtung erzielt. Im Falle eines im Querschnitt runden Gehäuses und eines runden Bauelements entspricht der Durchmesser des Innenraums des Gehäuses beispielsweise etwa dem dreifachen Durchmesser des optischen Bauelements.

In einer weiteren bevorzugten Ausgestaltung liegt ein Abstand der Außenkante des zumindest einen Bauelements von der Schwenkachse im Bereich zwischen etwa der Hälfte bis etwa zu drei Viertel einer Abmessung des Innenraums des Gehäuses von der Längsachse zur Innenwand des Gehäuses, vorzugsweise beträgt dieser Abstand etwa zwei Drittel der genannten Abmessung.

Diese Maßnahme hat den Vorteil, daß mit einem besonders kleinen Verstellweg eines etwaigen Stellgliedes eine sehr große Schwenkbewegung des zumindest einen Bauelements erzielt werden kann, wodurch das Umschalten der erfindungsgemäßen Vorrichtung zwischen der eingeschwenkten und der ausgeschwenkten Stellung des Bauelements ermöglicht wird. Im Falle eines im Querschnitt runden Gehäuses bedeutet dies, daß der Abstand des Mittelpunktes des zumindest einen Bauelements von der Schwenkachse im Bereich von etwa dem halben Radius bis etwa zu drei Viertel des Radius des Innenraums des Gehäuses von der Längsachse liegt, vorzugsweise bei etwa zwei Drittel des Innenradius des Gehäuses.

In einer weiteren bevorzugten Ausgestaltung sind zumindest zwei Bauelemente in dem Gehäuse angeordnet, und ist jedem dieser beiden Bauelemente ein separater Träger zugeordnet.

Während es auch denkbar wäre, zwei Bauelemente an nur einen Träger anzuordnen, deren Schwenkbewegungen dann zwangsläufig synchronisiert wären, hat diese Maßnahme den Vorteil, daß bei minimaler Baugröße des Gehäuses der Vorrichtung insgesamt drei Schaltzustände erreicht werden können, nämlich zwei Schaltzustände, bei denen wahlweise eines der beiden Bauelemente oder beide in den Strahlengang eingeschwenkt ist/sind, und einen dritten Schaltzustand, bei dem beide Bauelemente aus dem Strahlengang ausgeschwenkt sind.

Dabei ist es in einer bevorzugten Ausgestaltung vorgesehen, daß die Träger bezüglich der Längsachse an axial etwa gleicher Position angeordnet sind.

Hierbei ist von Vorteil, daß der bereits zuvor erwähnte Spalt, in dem das zumindest eine Bauelement in eingeschwenktem Zustand zu liegen kommt, nicht vergrößert werden muß, wodurch das optische System des endoskopischen Systems keine konstruktiven Einschränkungen erleidet.

In einer weiteren bevorzugten Ausgestaltung sind die Schwenkachsen der Träger in Umfangsrichtung des Gehäuses um etwa 90° zueinander versetzt.

Diese Maßnahme hat insbesondere zusammen mit der zuvor genannten Ausgestaltung den Vorteil, daß bei minimaler Baugröße des Gehäuses der Vorrichtung die zumindest zwei an axial etwa gleicher Position angeordneten Bauelemente in den Strahlengang einund ausgeschwenkt werden können und dabei insgesamt die bereits zuvor erwähnten drei Schaltzustände ermöglicht werden.

In einer weiteren bevorzugten Ausgestaltung sind die Träger bezüglich der Längsachse an axial unterschiedlichen Positionen angeordnet.

Diese Maßnahme hat den Vorteil, daß auch gleichzeitig zwei optische Bauelemente in den Strahlengang eingeschwenkt werden können, bspw. ein Filter und ein Polarisator, oder ein Filter und eine Blende, oder zwei Filter. Diese Maßnahme läßt sich nicht nur alternativ, sondern auch kumulativ mit den zuvor genannten Ausgestaltungen kombinieren, bspw. werden jeweils zwei Bauelemente mit ihren Trägern an axial gleicher Position angeordnet, während ein weiteres Paar von Bauelementen dann zu dem ersten Paar axial beabstandet angeordnet wird. Dadurch kann anstelle eines gegenüber einem einzelnen Bauelement vierfach so großen Spaltes ein doppelt so großer Spalt ausreichen, um die vier Bauelemente in den Strahlengang ein- bzw. auszuschwenken.

In einer weiteren bevorzugten Ausgestaltung ist zum Verschwenken des zumindest einen Bauelements ein von außerhalb des Gehäuses bedienbarer Betätigungsmechanismus vorgesehen, der zumindest ein Antriebselement aufweist, das mit zumindest einem am Träger angeordneten Mitnehmerelement zusammenwirkt, wobei der Abstand des zumindest einen Mitnehmerelements von der Schwenkachse klein gegen den Abstands eines Mittelpunkts des zumindest einen Bauelements von der Schwenkachse ist.

Diese Maßnahme, die durch die erfindungsgemäße Ausgestaltung der Vorrichtung im Unterschied zu dem bekannten Revolverrad ermöglicht wird, hat nun den Vorteil, daß für das zumindest eine Bauelement ein Betätigungsmechanismus vorgesehen wird, der dem Bediener einen sehr kurzen Betätigungsweg und damit eine sehr schnelle Umschaltung zwischen dem eingeschwenkten und dem ausgeschwenkten Zustand des zumindest einen Bauelements ermöglicht.

Dabei ist es weiterhin bevorzugt, wenn der Träger als zweiseitiger Hebel in bezug auf die Schwenkachse ausgebildet ist, wobei das zumindest eine Mitnehmerelement auf der dem zumindest einem Bauelement abgewandten Seite der Schwenkachse angeordnet ist.

Diese Maßnahme hat hinsichtlich der Konstruktion des Betätigungsmechanismus den Vorteil, daß das Mitnehmerelement und das Antriebselement in unmittelbarer Nähe der Gehäusewandung angeordnet werden können, um eine kraftschlüssige Verbindung oder Kraftübertragung zwischen dem zumindest einen Antriebselement und dem zumindest einem Mitnehmerelement zu erzielen. Eine kraftschlüssige Verbindung zwischen dem Antriebselement und dem Mitnehmerelement kann dabei in Form einer mechanischen Verbindung (Stift-Loch) bewirkt sein, oder es kann, wie in einer weiteren bevorzugten Ausgestaltung vorgesehen ist, eine magnetische Kraftübertragung in Form einer Magnetkupplung vorgesehen sein.

In einer weiteren bevorzugten Ausgestaltung weist der Betätigungsmechanismus ein Stellglied auf, an dem für jeden Träger zumindest ein Antriebselement vorgesehen ist, wobei die Antriebselemente mit den Mitnehmerelementen derart zusammenwirken, daß bei einer Verstellung des Verstellgliedes aus einer Ausgangsstellung, in der beide Bauelemente aus dem Strahlengang ausgeschwenkt sind, in eine erste Betriebsstellung, in der das eine Bauelement in den Strahlengang eingeschwenkt wird, und bei Verstellung des Stellgliedes aus der Ausgangsstellung in eine der ersten Betriebsstellung entgegengesetzte zweite Betriebsstellung das andere Bauelement in den Strahlengang eingeschwenkt wird.

Hierbei ist von Vorteil, daß nur ein Stellglied erforderlich ist, um die zumindest zwei optischen Bauelemente wahlweise in den Strahlengang ein- bzw. auszuschwenken, oder beide Bauelemente aus dem Strahlengang auszuschwenken. Hierdurch wird die Handhabung der erfindungsgemäßen Vorrichtung noch weiter verbessert, weil zwischen den Betriebszuständen der Vorrichtung noch schneller umgeschaltet werden kann.

In einer weiteren bevorzugten Ausgestaltung wird in der ersten und zweiten Betriebsstellung das jeweils ausgeschwenkte Bauelement im wesentlichen durch das Zusammenwirken des jeweiligen zumindest einen Antriebselements mit dem jeweiligen zumindest einen Mitnehmerelement in der ausgeschwenkten Stellung festgehalten.

Hierbei ist von Vorteil, daß keine die Störanfälligkeit des Betätigungsmechanismus erhöhenden Bauteile erforderlich sind, um die zumindest zwei Bauelemente in der eingeschwenkten oder ausgeschwenkten Stellung lagefest zu halten.

In einer weiteren bevorzugten Ausgestaltung ist das Stellglied in der Ausgangsstellung, der ersten und/oder der zweiten Betriebsstellung verrastbar.

Hierbei ist von Vorteil, daß nach dem Ein- bzw. Ausschwenken des zumindest einen oder der vorzugsweise zumindest zwei Bauelemente das Stellglied vom Benutzer losgelassen werden kann, ohne daß sich das Stellglied und damit das zumindest eine Bauelement in unerwünschter Weise selbsttätig, bspw. aufgrund von Schwerkraft, lageverstellt.

In einer weiteren bevorzugten Ausgestaltung ist das Stellglied in Umfangsrichtung des Gehäuses verstellbar und weist einen Bedienhebel auf.

Hierbei ist von Vorteil, daß die Bedienung bzw. Betätigung der erfindungsgemäßen Vorrichtung in sehr einfach zu handhabender, im wesentlichen ermüdungsfreier Weise ermöglicht wird.

In einer weiteren bevorzugten Ausgestaltung sind das zumindest eine Antriebselement und das zumindest eine Mitnehmerelement als magnetisch wirkende Elemente ausgebildet und wirken durch das Gehäuse hindurch magnetisch zusammen.

Diese Maßnahme hat den Vorteil, daß das Gehäuse der Vorrichtung insgesamt hermetisch dicht geschlossen ausgebildet werden kann, d.h. keine Öffnungen aufweist, die mittels Dichtungen verschlossen werden müssen, wodurch die erfindungsgemäße Vorrichtung sich für eine Sterilisation in einem Autoklaven eignet. Des weiteren kann die Vorrichtung auf diese Weise in das übrige Gehäuse eines endoskopischen Systems, bspw. eines Endoskops, integriert werden, wodurch das endoskopische System bzw. Endoskop als Ganzes mit einem hermetisch dicht geschlossenen Gehäuse ausgebildet werden kann.

Weitere Vorteile und Merkmale ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird mit Bezug auf diese hiernach näher beschrieben. Es zeigen:
- Fig. 1: eine Gesamtseitenansicht eines endoskopischen Systems in Form eines Endoskops;
- Fig. 2: den Ausschnitt A in Fig. 1 im Längsschnitt in gegenüber Fig. 1 vergrößertem Maßstab, wobei Fig. 2 ein Schnitt entlang der Linie II-II in Fig. 3 ist;
- Fig. 3: ein Schnitt entlang der Linie III-III in Fig. 2, wobei die Okularmuschel in Fig. 2 weggelassen wurde; und
- Figuren 4a) bis 4c): drei schematische Darstellungen dreier Betriebszustände einer in dem endoskopischen System in Fig. 1 verwendeten Vorrichtung zum Positionieren optischer Bauelemente in den endoskopischen System, wobei die Figuren 4a) bis c) einem Schnitt entlang der Linie IV-IV in Fig. 2 entsprechen.

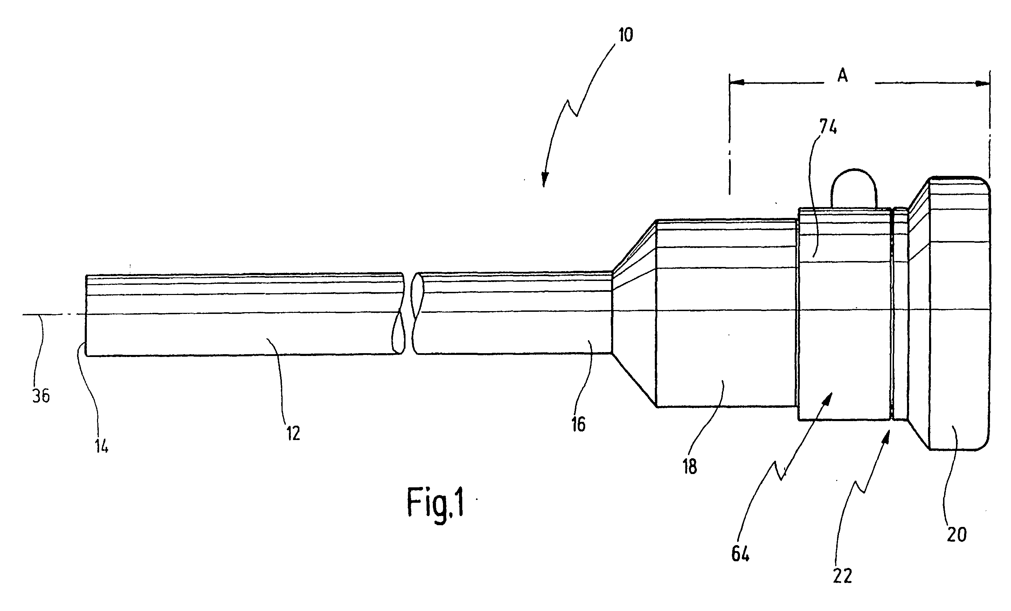

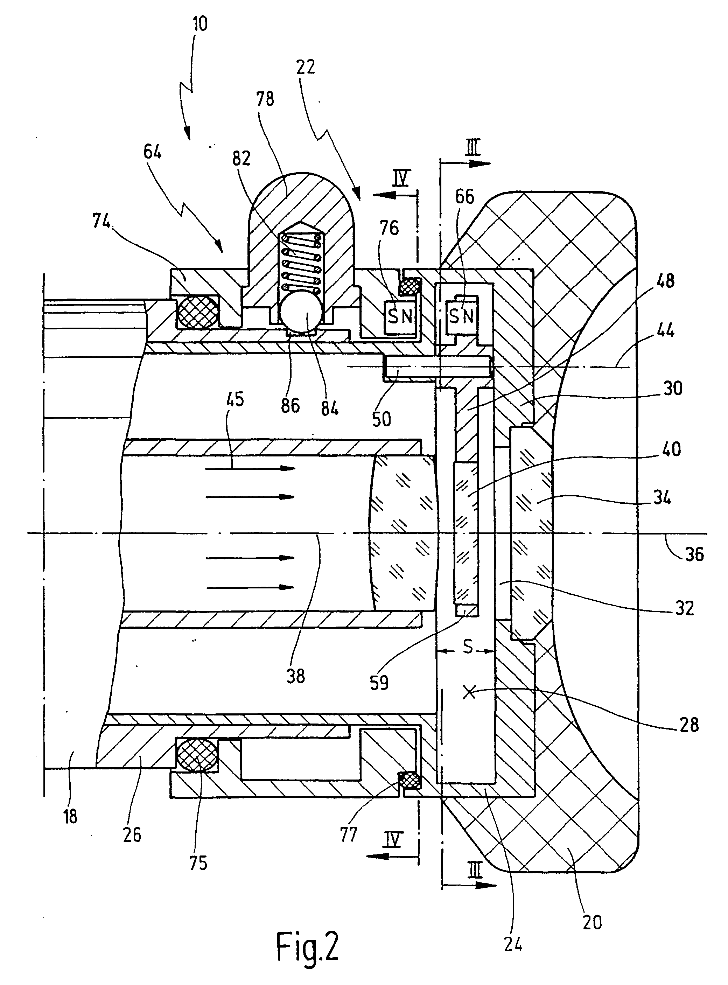

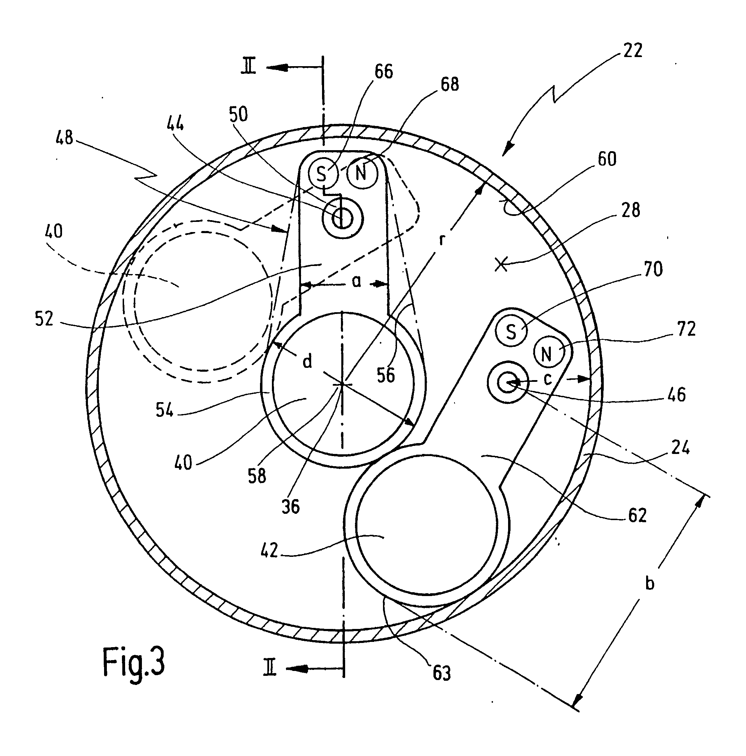

In Fig. 1 ist ein mit dem allgemeinen Bezugszeichen 10 versehenes Endoskop als endoskopischen System für die photodynamische Diagnose bzw. für die Fluoreszenzdiagnose dargestellt. Einzelheiten des Endoskops 10 sind in Figuren 2 und 3 dargestellt, auf die nachfolgend ebenfalls Bezug genommen wird.

Die Darstellung des Endoskops 10 in Fig. 1 ist schematisch und soll hier nur zur Erläuterung dienen.

Das Endoskop 10 weist einen langerstreckten Schaft 12 auf, in dem ein nicht näher dargestelltes optisches Abbildungssystem aus mehreren hintereinander angeordneten Linsen oder aus einem geordneten Lichtleitfaserbündel sowie ein ungeordnetes Faserbündel für die Beleuchtungslichtleitung enthalten ist. Ein distales Ende 14 des Schafts 12 bildet das lichteintrittsseitige Ende für Beobachtungslicht bzw. das lichtaustrittsseitige Ende für Beleuchtungslicht.

An ein proximales Ende 16 des Schafts 12 schließt sich ein Optikkopf 18 bzw. Okular an, der an seinem proximalen Ende eine Okularmuschel 20 aufweist.

Das Endoskop 10 weist eine mit dem allgemeinen Bezugszeichen 22 versehene Vorrichtung zum Positionieren zumindest eines optischen Bauelements innerhalb des Endoskops 10 auf, das hiernach mit Bezug auf Figuren 2 und 3 näher beschrieben wird.

Die Vorrichtung 22 weist ein Gehäuse 24 auf. Das Gehäuse 24 ist hier im Querschnitt rund, und ist mit einem ebenfalls im Querschnitt runden Gehäuse 26 des Optikkopfes 18 fest verbunden. Das Gehäuse 24 ist ferner hermetisch dicht abgeschlossen, d.h. ein Innenraum 28 des Gehäuses 24 ist gegen die Außenumgebung hermetisch dicht verschlossen. An seinem proximalen Abschnitt 30, an dem die Okularmuschel 20 befestigt ist, weist das Gehäuse 24 eine Lichtdurchtrittsöffnung 32 auf, die durch ein Deckglas 34 hermetisch dicht abgeschlossen ist. "Hermetisch dicht" bedeutet dabei, daß das Endoskop 10 in einem Autoklaven sterilisierbar ist, ohne daß Verunreinigungen oder Feuchtigkeit in den Innenraum 28 eindringen können.

Eine Längsachse 36 des Gehäuses 24, die hier die Längsmittelachse des Gehäuses bildet, und die auch die Längsmittelachse des Schaftes 12 bildet, fällt mit einer optischen Achse der Endoskopoptik des Endoskops 10 zusammen.

In dem Gehäuse 24 ist zumindest ein optisches Bauelement, und im vorliegenden Ausführungsbeispiel sind in dem Gehäuse zwei optische Bauelemente 40 und 42 angeordnet. Die optischen Bauelemente 40 und 42 sind bspw. zwei Filter für unterschiedliche Spektralbereiche.

Das optische Bauelement 40 ist um eine Schwenkachse 44, die im wesentlichen, hier genau parallel zur Längsachse 36 bzw. zur optischen Achse 38 verläuft, in den Strahlengang, der in Fig. 2 mit Pfeilen 45 angedeutet ist, einschwenkbar und aus dem Strahlengang wieder ausschwenkbar. In Figuren 2 und 3 ist das optische Bauelement 40 im in den Strahlengang eingeschwenkten Zustand dargestellt. Das optische Bauelement 42 ist dagegen aus dem Strahlengang ausgeschwenkt. Das optische Bauelement 42 ist um eine Schwenkachse 46, die ebenfalls parallel zur Längsachse 36 bzw. zur optischen Achse 38 verläuft, in den Strahlengang einschwenkbar und aus diesem ausschwenkbar.

Die Bauelemente 40 und 42 sind auf axial gleicher Position in bezug auf die Längsachse 36 angeordnet.

Die Schwenkachse 46 ist bezüglich der Schwenkachse 44 in Umfangsrichtung des Gehäuses 24 um etwa 90° versetzt angeordnet.

Das Bauelement 40 ist an einem Träger 48 angeordnet bzw. an diesem befestigt, der um die Schwenkachse 44 mittels eines Zapfens 50 am Gehäuse 24 der Vorrichtung 22 verschwenkbar gelagert ist.

Der Träger 48 weist einen ersten Abschnitt 52 sowie einen zweiten Abschnitt 54 auf. Der zweite Abschnitt 54 dient als Einfassung des optischen Bauelements 40 und wird in der vorliegenden Beschreibung als zu dem optischen Bauelement 40 gehörig angesehen. Es ist jedoch auch möglich, das Bauelement ohne den Abschnitt 54 am Abschnitt 52 zu befestigen, so daß unter dem Träger 48 nur der Abschnitt 52 verstanden wird.

Der Träger 48 ist insgesamt länglich ausgebildet und weist in Richtung der Längsachse 36, d.h. in axialer Richtung, eine Dicke auf, die etwa der Dicke des optischen Bauelements 40 entspricht. Der Träger 48 erstreckt sich im wesentlichen durchgehend gerade quer zur Längsachse 36.

Der Träger 48 weist in der Schwenkebene des optischen Bauelements 40 (Zeichenebene in Fig. 3) eine größte Abmessung a auf, die kleiner oder gleich einer größten Abmessung d des Bauelements 40 in der Schwenkebene ist. Die größte Abmessung d, d.h. im vorliegenden Fall der Ausgestaltung eines kreisförmigen Bauelements 40 der Durchmesser des Bauelements 40, versteht sich dabei im vorliegenden Fall auch einschließlich der zusätzlichen Abmessung des zweiten Abschnitts 54 des Trägers 48, der das Bauelement 40 einfaßt. Die Abmessung a des Trägers 48 kann also auch so groß sein wie der Durchmesser des Bauelements 40 einschließlich des zweiten Abschnittes 54. Diese Abmessung ist in Fig. 3 mit d bezeichnet. Während der Träger 48 im gezeigten Ausführungsbeispiel im wesentlichen die Form eines Rechteckes aufweist, kann der Träger sich auch bspw. bis auf den größten Durchmesser des zweiten Abschnittes 54 erweitern, wie mit einer strichpunktierten Linie 56 angedeutet ist. Für die Zwecke der Erfindung ist es ausreichend, wenn der Träger 48 auf derjenigen Seite, die beim Ausschwenken des Bauelements 40 aus dem Strahlengang sich dem Gehäuse 24 annähert, das optische Bauelement 40 nicht oder nicht wesentlich überragt, so daß der Träger 48 nicht am Gehäuse 24 in Anlage kommt, bevor das Bauelement 40 vollständig aus dem Strahlengang ausgeschwenkt ist.

Ein Abstand eines Mittelpunktes 58 des Bauelements 40, der im in den Strahlengang eingeschwenkten Zustand mit der Längsachse 36 bzw. mit der optischen Achse 38 zusammenfällt, bzw. einer Außenkante 59 von der Schwenkachse 44 ist größer als eine Hälfte der Abmessung r des Innenraums 28 des Gehäuses 24 von der Längsachse 36 zu einer Innenwand 60 des Gehäuses 24 in der Schwenkebene des Bauelements 40. In dem gezeigten Ausführungsbeispiel, in dem das Gehäuse 24 im Querschnitt rund ist, bedeutet dies, daß der Abstand des Mittelpunkts 58 des Bauelements 40 von der Schwenkachse 44 in der Schwenkebene des Bauelements 40 größer ist als der halbe Radius des Innenraums 28 des Gehäuses 24.

Der Abstand des Mittelpunkts 58 des Bauelements 40 von der Schwenkachse 44 liegt dabei vorzugsweise im Bereich zwischen etwa der Hälfte der Abmessung r, d.h. dem halben Radius des Innenraums 28 des Gehäuses 24 bis etwa zu drei Viertel der Abmessung r des Innenraums 28 des Gehäuses 24 von der Längsachse 36 zur Innenwand 60 des Gehäuses 24. Im gezeigten Ausführungsbeispiel liegt der Abstand des Mittelpunktes 58 des Bauelements 40 von der Schwenkachse 44 etwa bei zwei Drittel der Abmessung r.

Die Abmessung r des Innenraums 28 des Gehäuses 24 von der Längsachse 36 zur Innenwand 60 des Gehäuses 24 in der Schwenkebene des Bauelements 40 ist etwa 1,5 bis 2 Mal so groß wie die Abmessung d, d.h. der Durchmesser d des Bauelements 40 (im gezeigten Ausführungsbeispiel einschließlich der Abmessung des zweiten Abschnitts 54 des Trägers 48). Diese Wahl der Abmessung r des Innenraums 28 des Gehäuses 24 ist somit gerade ausreichend, daß das Bauelement 40 vollständig aus dem Strahlengang ausgeschwenkt werden kann, wie in Fig. 3 mit unterbrochenen Linien des Bauelements 40 angedeutet ist. Des weiteren ermöglicht es diese Wahl der Abmessung r des Innenraums 28 des Gehäuses 24, das zweite Bauelement 42 auf axial gleicher Position wie das optische Bauelement 40 in dem Gehäuse 24 anzuordnen, und es auch diesem Bauelement 42 zu ermöglichen, vollständig aus dem Strahlengang ausgeschwenkt zu werden, bei gleichzeitig minimaler Gehäuseabmessung.

Das Bauelement 42 ist an einem separaten Träger 62 angeordnet, der im gezeigten Ausführungsbeispiel identisch mit dem Träger 48 ausgebildet ist und demnach hier nicht näher beschrieben werden muß. Der Träger 62 liegt wie die Bauelemente 40 und 42 in bezug auf die Längsachse 36 auf axial gleicher Position wie der Träger 48, wobei lediglich die Schwenkachse 46 des Trägers 62 von der Schwenkachse 44 des Trägers 48 in Umfangsrichtung des Gehäuses 24 um etwa 90° versetzt angeordnet ist.

Wie für das Bauelement 42 mit dem Träger 62 in Fig. 3 dargestellt ist, während das gleiche für das Bauelement 40 und den Träger 48 gilt, ist ein kleinster Abstand c der Schwenkachse 46 von der Innenwand 60 des Gehäuses 24 kleiner als ein größter Abstand b der Schwenkachse 46 zu einer hier umfänglichen Außenkante 63 des Bauelements 42.

Aufgrund der Anordnung der Bauelemente 40 und 42 kann jeweils eines der beiden Bauelemente 40 und 42 wahlweise in den Strahlengang eingeschwenkt werden, wie später noch näher beschrieben wird. Denkbar ist es jedoch auch, die Bauelemente 40 und 42 an axial unterschiedlichen Positionen anzuordnen, so daß beide Bauelemente dann gleichzeitig in den Strahlengang eingeschwenkt werden können, und dabei mehr als zwei Bauelemente vorzusehen.

Wie in Fig. 2 dargestellt ist, kann ein Spalt s in Richtung der Längsachse 36, der zur Positionierung der Bauelemente 40 und 42 in den Strahlengang vorgesehen ist, so klein gewählt werden, daß der Spalt s im wesentlichen der axialen Abmessung bzw. Dicke der Bauelemente 40 und 42 entspricht. Somit baut die Vorrichtung 22 nicht nur quer zur Längsachse 36, sondern auch in Richtung der Längsachse 36, d.h. axial, sehr klein.

Mit Bezug auf Figuren 1 bis 4 wird nachfolgend ein Betätigungsmechanismus 64 zum Ein- und Ausschwenken der Bauelemente 40 und 42 in und aus dem Strahlengang des Endoskops 10 näher beschrieben.

Am Träger 28 ist zumindest ein und sind im gezeigten Ausführungsbeispiel zwei Mitnehmerelemente 66 und 68 in Form magnetisch wirkender Elemente, bspw. kleiner Magnete, angeordnet, die entgegengesetzt polarisiert sind. Mit S ist der magnetische Südpol und mit N der magnetische Nordpol bezeichnet.

Der Abstand jedes Mitnehmerelements 66 und 68 von der Schwenkachse 44 ist dabei klein gegenüber dem Abstand des Mittelpunktes 58 des Bauelements 40 von der Schwenkachse 44. Der Träger 48 ist somit als zweiseitiger, hier gerader Hebel ausgebildet, dessen einer Hebelarm durch die Strecke zwischen den Mitnehmerelementen 66, 68 und der Schwenkachse 44, und dessen anderer Hebelarm durch die Strecke zwischen der Schwenkachse 44 und dem Mittelpunkt 58 des Bauelements 40 gebildet wird. Das Verhältnis der Hebelarmlängen beträgt im gezeigten Ausführungsbeispiel etwa 1:4.

An dem Träger 62 sind entsprechende Mitnehmerelemente 70 und 72 angeordnet, die bezüglich der Schwenkachse 46 gleich positioniert sind wie die Mitnehmerelemente 66 und 68 in bezug auf die Schwenkachse 44.

Die Mitnehmerelemente 66, 68 sind auf der dem Bauelement 40 abgewandten Seite der Schwenkachse 44 angeordnet, ebenso wie die Mitnehmerelemente 70 und 72 auf der dem Bauelement 42 gegenüberliegenden Seite der Schwenkachse 46 am Träger 62 angeordnet sind.

Der Betätigungsmechanismus 64 weist ein Stellglied 74 in Form eines das Gehäuse 24 umgebenden Stellringes auf, der mittels O-Ringen 75 und 77 gehemmt ist, um einen für die Handhabung günstigen Verstellwiderstand zu erzeugen.

An dem Stellglied 74 ist eine Mehrzahl von Antriebselementen 76 bzw. 79 angeordnet. Dabei sind vier Antriebselemente 79 den Mitnehmerelementen 66 und 68 des Trägers 48 zugeordnet, während vier Antriebselemente 79 den Mitnehmerelementen 70 und 72 des Trägers 62 zugeordnet sind. Die Antriebselemente 76 und 79 sind fest mit dem Stellglied 74 verbunden. Das Stellglied 74 läßt sich in Umfangsrichtung des Gehäuses 24 in beiden Drehrichtungen um die Längsachse 36 verstellen.

Die Antriebselemente 76 und 79 sind ebenfalls als magnetisch wirkende Elemente, bspw. kleine Magnete ausgebildet und wirken mit den Mitnehmerelementen 66, 68 bzw. 70, 72 durch das Gehäuse 24 hindurch magnetisch zusammen.

Die Polarisierung bzw. die Pole der Antriebselemente 76 und 79 sind wiederum mit S und N bezeichnet.

Das Stellglied 74 weist zur Verstellung in Umfangsrichtung des Gehäuses 24 einen Bedienhebel 78 auf, der bspw. mit dem Daumen betätigbar ist.

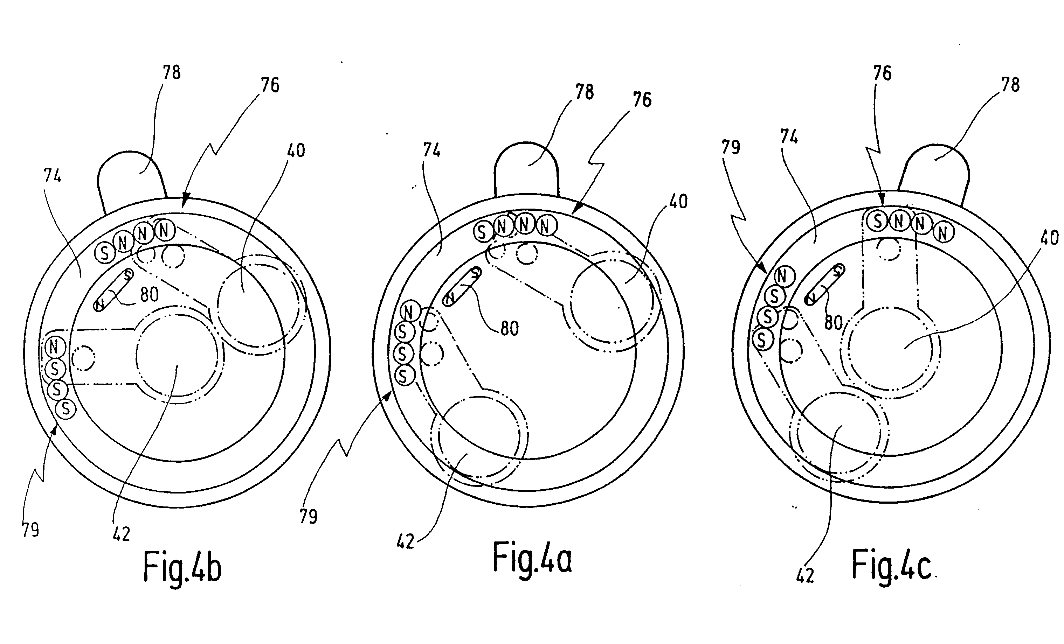

Wie in Fig. 4a bis c) dargestellt ist, ermöglicht der Betätigungsmechanismus 64 drei Betriebsstellungen der Vorrichtung 22.

In Fig. 4a) ist eine Ausgangsstellung dargestellt, in der beide Bauelemente 40 und 42 aus dem Strahlengang ausgeschwenkt sind. Die Bauelemente 40 und 42 werden in dieser Ausgangsstellung im wesentlichen allein durch die magnetische Wechselwirkung zwischen den Antriebselementen 76 und 79 und den Mitnehmerelementen 66, 68 bzw. 70, 72 in der ausgeschwenkten Stellung gehalten. Zur weiteren Lagefixierung ist in dem Gehäuse noch ein lagefester Zusatzmagnet 80 optional vorgesehen.

Wird nun ausgehend von Fig. 4a) das Stellglied 74 entgegen dem Uhrzeigersinn (Fig. 4b) verstellt, wird das Bauelement 42 durch den magnetischen Mitnahmeeffekt zwischen den Antriebselementen 79 und den Mitnehmerelementen 70 und 72 in den Strahlengang eingeschwenkt, während das Bauelement 40 aufgrund der magnetischen Wechselwirkung zwischen den Mitnehmerelementen 66 und 68 und den diesen zugeordneten Antriebselementen 76 in der ausgeschwenkten Stellung gehalten wird. Wie aus einem Vergleich der Fig. 4a) und 4b) ersichtlich ist, ist der Verstellweg des Stellgliedes 74 zum Umschalten bzw. Einschwenken des Bauelements 42 in den Strahlengang sehr gering, d.h. wesentlich geringer als der von dem Bauelement 42 beim Umklappen zurückgelegte Weg.

Ausgehend von Fig. 4b) wird das Bauelement 42 wieder aus dem Strahlengang ausgeschwenkt, indem das Stellglied 74 im Uhrzeigersinn wieder in die in Fig. 4a) dargestellte Ausgangsstellung verstellt wird. Ausgehend von der in Fig. 4a) dargestellten Ausgangstellung kann nun durch Verstellen des Stellgliedes 74 im Uhrzeigersinn in die Fig. 4c) dargestellte zweite Betriebsstellung, die der ersten Betriebsstellung entgegengesetzt ist, das Bauelement 40 in den Strahlengang eingeschwenkt werden, während das Bauelement 42 wiederum aufgrund der magnetischen Wechselwirkung zwischen den Mitnehmerelementen 70, 72 und den zugeordneten Antriebselementen 79, ggf. mit Unterstützung des Zusatzmagneten 80, in der ausgeschwenkten Stellung gehalten wird. Durch Zurückbewegen des Stellgliedes 74 aus der in Fig. 4c) dargestellten zweiten Betriebsstellung in die in Fig. 4a) dargestellte Ausgangsstellung wird das Bauelement 40 wieder aus dem Strahlengang ausgeschwenkt.

Das Stellglied 74 ist in der Ausgangsstellung, der ersten und zweiten Betriebsstellung verrastbar. Dazu ist der Bedienhebel 78 mit einer Raste in Form einer mittels einer Feder 82 belasteten Kugel 84 ausgestattet, wobei in dem Gehäuse 26 des Endoskops 10 gemäß der Ausgangsstellung, ersten und zweiten Betriebsstellung des Stellgliedes 74 umfänglich verteilt drei Aussparungen 86 vorgesehen sind, in die die Kugel 84 jeweils einspringt.

Wie aus Fig. 4a) bis c) hervorgeht, sind die Antriebselemente 76 und 79 in Form der Magnete hinsichtlich der Polarisierung der Magnete so gewählt, daß sie mit den entsprechenden Mitnehmerelementen 66, 68 bzw. 70, 72 in der jeweils eingeschwenkten Stellung des Bauelements 40 bzw. 42 einen vollständigen Kraftschluß besitzen, während die Antriebselemente 76 im jeweils ausgeschwenkten Zustand der Bauelemente 40 und 42 mit den jeweiligen Mitnehmerelementen 66, 68, bzw. 70, 72 so zusammenwirken, daß sie auf die jeweiligen Träger 48 bzw. 62 ein Drehmoment zur ausgeschwenkten Stellung hin ausüben, wodurch die Bauelement 40 und 42 in ihrer ausgeschwenkten Stellung festgehalten werden.

## Patentansprüche

1. Vorrichtung zum Positionieren zumindest eines optischen Bauelements (40, 42) innerhalb eines endoskopischen Systems, mit einem Gehäuse (24), durch das eine optische Achse (38) des endoskopischen Systems verläuft, und in dem zumindest ein Bauelement (40, 42) angeordnet ist, das um eine im wesentlichen parallel zu einer Längsachse (36) des Gehäuses (24) verlaufende Schwenkachse (44, 46) in den Strahlengang einschwenkbar und aus dem Strahlengang wieder ausschwenkbar ist, wobei das zumindest eine Bauelement (40, 42) an einem um die Schwenkachse (44, 46) verschwenkbaren Träger (48, 62) angeordnet ist, **dadurch gekennzeichnet, daß** der kleinste Abstand (c) einer Innenwand (60) des Gehäuses (24) von der Schwenkachse (44, 46) kleiner ist als der größte Abstand (b) der Schwenkachse (44, 46) zu einer Außenkante (63) des zumindest einen Bauelements (40, 42).

2. Vorrichtung nach Anspruch 1, **dadurch gekennzeichnet, daß** der Radius (r) des Innenraums (28) des Gehäuses (24) von der Längsachse (36) zur Innenwand (60) des Gehäuses (24) in der Schwenkebene des zumindest einen Bauelements (40, 42) etwa 1,5 bis 2 Mal so groß ist wie die größte Abmessung (d) des zumindest einen Bauelements (40, 42) in der Schwenkebene.

3. Vorrichtung nach Anspruch 1 oder 2, **dadurch gekennzeichnet, daß** der Abstand der Außenkante (63) des zumindest einen Bauelements (40, 42) von der Schwenkachse (36) im Bereich zwischen etwa der Hälfte bis etwa zu drei Viertel des Radius (r) des Innenraums (28) des Gehäuses (24) von der Längsachse (36) zur Innenwand (60) des Gehäuses. liegt, vorzugsweise etwa zwei Drittel dieser Abmessung (r) beträgt.

4. Vorrichtung nach einem der Ansprüche 1 bis 3, **dadurch gekennzeichnet, daß** zumindest zwei Bauelemente (40, 42) in dem Gehäuse (24) angeordnet sind, und daß jedem Bauelement (40, 42) ein separater Träger (48, 62) zugeordnet ist.

5. Vorrichtung nach Anspruch 4, **dadurch gekennzeichnet, daß** die Träger (48, 62) bezüglich der Längsachse (36) an axial etwa gleicher Position angeordnet sind.

6. Vorrichtung nach Anspruch 4 oder 5, **dadurch gekennzeichnet, daß** die Schwenkachsen (44, 46) der Träger (48, 62) in Umfangsrichtung des Gehäuses um etwa 90° zueinander versetzt sind.

7. Vorrichtung nach einem der Ansprüche 4 bis 6, **dadurch gekennzeichnet, daß** die Träger (48, 62) bezüglich der Längsachse (36) an axial unterschiedlichen Positionen angeordnet sind.

8. Vorrichtung nach einem der Ansprüche 1 bis 7, **dadurch gekennzeichnet, daß** zum Verschwenken des zumindest einen Bauelements (40, 42) ein von außerhalb des Gehäuses (24) bedienbarer Betätigungsmechanismus (64) vorgesehen ist, der zumindest ein Antriebselement (76, 79) aufweist, das mit zumindest einem am Träger angeordneten Mitnehmerelement (66, 68, 70, 72) zusammenwirkt, wobei der Abstand des zumindest einen Mitnehmerelements (66, 68, 70, 72) von der Schwenkachse (44, 46) klein gegen den Abstand des Mittelpunkts (58) des zumindest einen Bauelements (40, 42) von der Schwenkachse (44, 46) ist.

9. Vorrichtung nach Anspruch 8, **dadurch gekennzeichnet, daß** der Träger (48, 62) als zweiseitiger Hebel in bezug auf die Schwenkachse (44, 46) ausgebildet ist, wobei das zumindest eine Mitnehmerelement (66, 68, 70, 72) auf der dem zumindest einen Bauelement (40, 42) abgewandten Seite der Schwenkachse (44, 46) angeordnet ist.

10. Vorrichtung nach einem der Ansprüche 4 bis 7 und nach Anspruch 8 oder 9, **dadurch gekennzeichnet, daß** der Betätigungsmechanismus (64) ein Stellglied (74) aufweist, an dem für jeden Träger (48, 62) zumindest ein Antriebselement (76) vorgesehen ist, wobei die Antriebselemente (76, 79) mit den Mitnehmerelementen (66, 68, 70, 72) derart zusammenwirken, daß bei einer Verstellung des Stellgliedes (74) aus einer Ausgangsstellung, in der beide Bauelemente (40, 42) aus dem Strahlengang ausgeschwenkt sind, in eine erste Betriebsstellung das eine Bauelement (40, 42) in den Strahlengang eingeschwenkt wird, und bei Verstellung des Stellgliedes (74) aus der Ausgangsstellung in eine der ersten Betriebsstellung entgegengesetzte zweite Betriebsstellung das andere Bauelement (40, 42) in den Strahlengang eingeschwenkt wird.

11. Vorrichtung nach Anspruch 10, **dadurch gekennzeichnet, daß** in der ersten und zweiten Betriebsstellung das jeweils ausgeschwenkte Bauelement (40, 42) im wesentlichen durch das Zusammenwirken des jeweiligen zumindest einen Antriebselements (76, 79) mit dem jeweiligen zumindest einen Mitnehmerelement (66, 68, 70, 72) in der ausgeschwenkten Stellung festgehalten wird.

12. Vorrichtung nach Anspruch 10 oder 11, **dadurch gekennzeichnet, daß** das Stellglied (74) in der Ausgangsstellung, der ersten und/oder der zweiten Betriebsstellung verrastbar ist.

13. Vorrichtung nach einem der Ansprüche 10 bis 12, **dadurch gekennzeichnet, daß** das Stellglied (74) in Umfangsrichtung des Gehäuses verstellbar ist und einen Bedienhebel aufweist.

14. Vorrichtung nach einem der Ansprüche 8 bis 13, **dadurch gekennzeichnet, daß** das zumindest eine Antriebselement (76) und das zumindest eine Mitnehmerelement (66, 68, 70, 72) als magnetisch wirkende Elemente ausgebildet sind und durch das Gehäuse (24) hindurch magnetisch zusammenwirken.

## Claims

1. A device for positioning at least one optical component (40, 42) inside an endoscopic system, comprising a housing (24) through which an optical axis (38) of the endoscopic system extends and in which at least one component (40, 42) is arranged, which component (40, 42) can be pivoted into the beam path and back out of the beam path about a pivot axis (44, 46) extending substantially parallel to a longitudinal axis (36) of the housing (24), said at least one component (40, 42) being arranged on a carrier (48, 62) which is pivotable about the pivot axis (44, 46), **characterized in that** the smallest distance (c) of an inside wall (60) of the housing (24) from the pivot axis (44, 46) is smaller than the greatest distance (b) of the pivot axis (44, 46) to an outer edge (63) of the at least one component (40, 42).

2. The device of claim 1, **characterized in that** the radius (r) of the interior (28) of the housing (24) from the longitudinal axis (36) to the inside wall (60) of the housing (24) in the pivot plane of the at least one component (40, 42) is about 1.5 to 2 times as great as the greatest dimension (d) of the at least one component (40, 42) in the pivot plane.

3. The device of claim 1 or 2, **characterized in that** the distance of the outer edge (63) of the at least one component (40, 42) from the pivot axis (36) is in the range of between about a half to about three quarters of the radius (r) of the interior (28) of the housing (24) from the longitudinal axis (36) to the inside wall (60) of the housing, preferably about two thirds of this dimension (r).

4. The device of anyone of claims 1 through 3, **characterized in that** at least two components (40, 42) are arranged in the housing (24), and **in that** each component (40, 42) is assigned a separate carrier (48, 62).

5. The device of claim 4, **characterized in that** the carriers (48, 62) are arranged at axially about the same position relative to the longitudinal axis (36).

6. The device of claim 4 or 5, **characterized in that** the pivot axes (44, 46) of the carriers (48, 62) are offset by approximately 90° with respect to one another in the circumferential direction of the housing.

7. The device of anyone of claims 4 through 6, **characterized in that** the carriers (48, 62) are arranged at axially different positions relative to the longitudinal axis (36).

8. The device of anyone of claims 1 through 7, **characterized in that**, in order to pivot the at least one component (40, 42), an actuating mechanism (64) is provided which can be operated from outside the housing (24) and which has at least one driver element (76, 79) cooperating with at least one driven element (66, 68, 70, 72) arranged on the carrier, in which case the distance of the at least one driver element (66, 68, 70, 72) from the pivot axis (44, 46) is small compared to the distance of the midpoint (58) of the at least one component (40, 42) from the pivot axis (44, 46).

9. The device of claim 8, **characterized in that** the carrier (48, 62) is designed as a two-sided lever in relation to the pivot axis (44, 46), the at least one driven element (66, 68, 70, 72) being arranged on that side of the pivot axis (44, 46) directed away from the at least one component (40, 42).

10. The device of anyone of claims 4 through 7 and of claim 8 or 9, **characterized in that** the actuating mechanism (64) has an adjustment member (74) on which at least one driver element (76) is provided for each carrier (48, 62), the driver elements (76, 79) cooperating with the driven elements (66, 68, 70, 72) in such a way that, when the adjustment member (74) is moved from a starting position, in which both components (40, 42) are pivoted out from the beam path, to a first operating position, one component (40, 42) is pivoted into the beam path, and, when the adjustment member (74) is moved from the starting position to a second operating position opposite to the first operating position, the other component (40, 42) is pivoted into the beam path.

11. The device of claim 10, **characterized in that**, in the first and second operating positions, the component (40, 42) respectively pivoted out is held securely in the pivoted-out position mainly by the interaction of the respective at least one driver element (76, 79) and the respective at least one driven element (66, 68, 70, 72).

12. The device of claim 10 or 11, **characterized in that** the adjustment member (74) can be locked in the starting position and the first and/or the second operating position.

13. The device of anyone of claims 10 through 12, **characterized in that** the adjustment member (74) is adjustable in the circumferential direction of the housing and has an operating lever.

14. The device of anyone of claims 8 through 13, **characterized in that** the at least one driver element (76) and the at least one driven element (66, 68, 70, 72) are designed as magnetically acting elements and interact magnetically through the housing (24).

## Revendications

1. Dispositif pour le positionnement d'au moins un composant (40, 42) optique à l'intérieur d'un système endoscopique, comprenant un boîtier (24), par lequel passe un axe optique (38) du système endoscopique, et dans lequel est disposé au moins un composant (40, 42), qui peut être basculé dans la trajectoire du faisceau autour d'un axe de pivotement (44, 46) disposé sensiblement parallèlement à un axe longitudinal (36) du boîtier (24) et peut être ressorti par basculement de la trajectoire du faisceau, le au moins un composant (40, 42) étant disposé sur un support (48, 62) pouvant basculer autour de l'axe de pivotement (44, 46), **caractérisé en ce que** la plus petite distance (c) d'une paroi intérieure (60) du boîtier (24) à l'axe de pivotement (44, 46) est inférieure à la plus grande distance (b) de l'axe de pivotement (44, 46) à une arête extérieure (63) du au moins un élément (40, 42).

2. Dispositif selon la revendication 1, **caractérisé en ce que** le rayon (r) de l'espace intérieur (28) du boîtier (24) de l'axe longitudinal (36) à la paroi interne (60) du boîtier (24) dans le plan de pivotement du au moins un composant (40, 42) est à peu près 1,5 à 2 fois aussi grand que la dimension (d) maximale du au moins un composant (40, 42) dans le plan de pivotement.

3. Dispositif selon la revendication 1 ou 2, **caractérisé en ce que** la distance du bord extérieur (63) du au moins un composant (40, 42) à l'axe de pivotement (36) se situe dans la plage entre à peu près la moitié jusqu'à environ trois-quarts du rayon (r) de l'espace intérieur (28) du boîtier (24) de l'axe longitudinal (36) à la paroi intérieure (60) du boîtier, et s'élève de préférence à environ deux tiers de cette dimension (r).

4. Dispositif selon l'une quelconque des revendications 1 à 3, **caractérisé en ce qu'**au moins deux composants (40, 42) sont disposés dans le boîtier (24) et **en ce qu'**un support (48, 62) séparé est attribué à chaque composant (40, 42).

5. Dispositif selon la revendication 4, **caractérisé en ce que** les supports (48, 62) sont disposés par rapport à l'axe longitudinal (36) en une position à peu près identique au plan axial.

6. Dispositif selon la revendication 4 ou 5, **caractérisé en ce que** les axes de basculement (44, 46) des supports (48, 62) sont décalés d'environ 90° l'un par rapport à l'autre dans le sens périphérique du boîtier.

7. Dispositif selon l'une quelconque des revendications 4 à 6, **caractérisé en ce que** les supports (48, 62) sont disposés par rapport à l'axe longitudinal (36) en des positions différentes sur un plan axial.

8. Dispositif selon l'une quelconque des revendications 1 à 7, **caractérisé en ce que**, pour le basculement du au moins un composant (40, 42), il est prévu un mécanisme d'actionnement (64) pouvant être commandé à l'extérieur du boîtier (24), qui présente au moins un élément d'entraînement (76, 79), qui coopère avec au moins un élément entraîneur (66, 68, 70, 72) disposé sur le support, la distance du au moins un élément entraîneur (66, 68, 70, 72) à l'axe de pivotement (44, 46) étant faible par rapport à la distance du centre (58) du au moins un composant (40, 42) à l'axe de pivotement (44, 46).

9. Dispositif selon la revendication 8 **caractérisé en ce que** le support (48, 62) est conçu comme un levier à deux côtés par rapport à l'axe pivotement (44, 46), le au moins un élément entraîneur (66, 68, 70, 72) étant disposé sur le côté, opposé à au moins un composant (40, 42), de l'axe de basculement (44, 46).

10. Dispositif selon l'une quelconque des revendications 4 à 7 et selon la revendication 8 ou 9, **caractérisé en ce que** le mécanisme d'actionnement (64) présente un actionneur (74), sur lequel au moins un élément d'entraînement (76) est prévu pour chaque support (48, 62), les éléments d'entraînement (76, 79) coopérant avec les éléments entraîneurs (66, 68, 70, 72) de telle sorte que, lors d'un déplacement de l'actionneur (74) à partir d'une position de départ, dans laquelle les deux composants (40, 42) sont sortis de la trajectoire du faisceau par basculement vers l'extérieur, dans une première position de service, un composant (40, 42) est basculé vers l'intérieur dans la trajectoire de faisceau, et en cas de déplacement de l'actionneur (74) à partir de la position de départ dans une seconde position de service opposée à la première position de service, l'autre composant (40, 42) est basculé vers l'intérieur dans la trajectoire du faisceau.

11. Dispositif selon la revendication 10, **caractérisé en ce que**, dans la première et la seconde positions de service, le composant (40, 42) respectivement basculé à l'extérieur est maintenu dans la position basculée vers l'extérieur essentiellement par l'action conjuguée du au moins un élément d'entraînement (76, 79) respectif avec le au moins un élément entraîneur (66, 68, 70, 72) respectif.

12. Dispositif selon la revendication 10 ou 11, **caractérisé en ce que** l'actionneur (74) peut être encliqueté dans la position de départ, la première et/ou la seconde position(s) de service.

13. Dispositif selon l'une quelconque des revendications 10 à 12, **caractérisé en ce que** l'actionneur (74) est déplaçable dans le sens périphérique du boîtier et présente un levier de commande.

14. Dispositif selon l'une quelconque des revendications 8 à 13, **caractérisé en ce que** le au moins un élément d'entraînement (76) et le au moins un élément entraîneur (66, 68, 70, 72) sont conçus comme des éléments agissant de façon magnétique et sont en interaction sur un plan magnétique à travers le boîtier (24).
